(19) Europäisches Patentamt / European Patent Office / Office européen des brevets

(11) **EP 4 114 355 B1**

(12) **EUROPEAN PATENT SPECIFICATION**

(45) Date of publication and mention
of the grant of the patent:
**10.07.2024 Bulletin 2024/28**

(21) Application number: **21708226.2**

(22) Date of filing: **01.03.2021**

(51) International Patent Classification (IPC):
*A61K 9/00* (2006.01)  *A61K 8/14* (2006.01)
*A61K 9/127* (2006.01)  *A61K 31/352* (2006.01)
*A61K 47/14* (2017.01)  *A61K 47/36* (2006.01)

(52) Cooperative Patent Classification (CPC):
**A61K 9/0014; A61K 8/14; A61K 8/345;
A61K 8/347; A61K 8/39; A61K 8/73; A61K 9/1272;
A61K 9/1277; A61K 31/05; A61K 47/14;
A61K 47/36; A61Q 17/00; A61Q 19/00;**
A61K 2800/75; A61Q 19/08

(86) International application number:
**PCT/EP2021/055003**

(87) International publication number:
**WO 2021/175763 (10.09.2021 Gazette 2021/36)**

(54) **TOPICAL COMPOSITION COMPRISING CANNABIDIOL**

TOPISCHE ZUSAMMENSETZUNG MIT CANNABIDIOL

COMPOSITION TOPIQUE COMPRENANT DU CANNABIDIOL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Validation States:
**MA**

(30) Priority: **03.03.2020 IT 202000004450**

(43) Date of publication of application:
**11.01.2023 Bulletin 2023/02**

(73) Proprietor: **Curaleaf International Limited
London EC2A 2EW (GB)**

(72) Inventors:
• **PACCHETTI, Barbara
6600 LOCARNO (CH)**
• **COSTANZO, Antonio
LONDON W12 8PJ (GB)**

(74) Representative: **PGA S.p.A.
Via Mascheroni, 31
20145 Milano (IT)**

(56) References cited:
**WO-A1-2019/023668      US-A1- 2017 281 481**

• **RIPAMONTI EMILIANO ET AL: "Evaluation of the
efficacy of proprietary niosomes as enhancers of
skin permeability of plant extracts: an in vitro
study", H&PC TODAY - HOUSEHOLD AND
PERSONAL CARE TODAY, vol. 13, no. 2, 1 March
2018 (2018-03-01), pages 14 - 17, XP093125165**
• **SIHORKAR V ET AL: "POLYSACCHARIDE
COATED NIOSOMES FOR ORAL DRUG
DELIVERY: FORMULATION AND IN VITRO
STABILITY STUDIES", PHARMAZIE, GOVI
VERLAG PHARMAZEUTISCHER VERLAG GMBH,
DE, vol. 55, no. 2, 1 February 2000 (2000-02-01),
pages 107 - 113, XP000890603, ISSN: 0031-7144**

## Description

### FIELD OF THE INVENTION

The present invention relates to a topical composition comprising cannabinoids. In particular, the present invention relates to a topical composition comprising cannabidiol incorporated into niosomes, preferably having size lower than 500 nm, and at least one topically acceptable excipient.

### STATE OF THE ART

Cannabis sativa L. is an annual herbaceous plant belonging to the Cannabaceae family, known since ancient times for its industrial and therapeutic uses.

Cannabis inflorescences or *flos* contain the highest concentration of cannabinoids, the characteristic and exclusive actives of the Cannabis plant, the two main ones being delta-9-tetrahydrocannabinol (THC) and cannabidiol (CBD).

Currently, more than 500 substances have been separated from hemp plants, among which are more than 120 cannabinoids, e.g. tetrahydrocannabinol (THC), cannabidiol (CBD), cannabichromene (CBC), cannabigerol (CBG), cannabidivarin (CBDV), cannabinol (CBN) and others, which are present in varying amounts in the aerial parts of the hemp plant.

Cannabis and its derivatives are currently accredited for the treatment of a number of pathological conditions, including chemotherapy-induced vomiting, nausea and pain, spasticity associated with multiple sclerosis and some epilepsies (Abrams, D.I., European Journal of Internal Medicine, March 2018, Volume 49, Pages 7-11). Δ9-THC is one of the main cannabinoids in Cannabis responsible for its therapeutic effects and uses, but its psychoactive potential and addictive nature have limited its therapeutic development. Cannabidiol (CBD) is the other main cannabinoid present in the cannabis plant which, unlike Δ9-THC, is devoid of psychotropic activity, devoid of cannabimimetic effect and devoid of addictive potential (Babalonis et al., Drug Alcohol Depend. 2017 March 01; 172: 9-13). The chemistry and pharmacology of CBD, as well as its molecular targets, have recently been reviewed. Indeed, long neglected, CBD is currently gaining attention for its therapeutic properties (Pisanti et al., Cannabidiol: State of the art and new challenges for therapeutic applications, Pharmacology and Therapeutics, Volume 175, July 2017, Pages 133-150) including skin disorders.

Cannabidiol is predominantly extracted from the aerial parts of cannabis (Cannabis sativa L.), but is also produced synthetically. Cannabidiol may have beneficial effects on the skin and interact with the skin's endocannabinoid system, for example by regulating sebum secretion. Numerous scientific studies have confirmed the regenerative abilities of cannabidiol, which is also useful as an anti-inflammatory, antioxidant, healing and antibacterial agent for skin and skin appendages. Cannabidiol can also support natural cell renewal and acts as a protective shield against harmful environmental influences (anti-pollution effect).

However, cannabidiol is also known to be unsuitable for the preparation of topical formulations, particularly cosmetic products and/or medical devices and/or pharmaceutical preparations for application to skin and mucous membranes, due to its poor solubility in water, and its consequent instability or incompatibility with the high percentage of water usually present in a cream, lotion, gel or other topical formulation. Cannabidiol is therefore currently used in oily solutions/suspensions, as for example the composition described in WO2019/023668 A1, comprising cannabidiol in a mixture of fatty acids, with a poor ability to penetrate through the skin and epithelia, which prevents or reduces the effectiveness of topical cannabidiol administration.

The use of niosomes to improve solubility and stability in topical formulations and penetration through the skin of cannabidiol has been suggested by E. Ripamonti et al, "Evaluation of the efficacy of proprietary niosomes as enhancers of skin permeability of plant extracts: an in vitro study", H&PC Today, vol. 13(2) March/April 2018, but results obtained with a niosome model described in WO 2017/168354 A1 did not yield statistically significant results.

To date, the cosmetic industry is still searching for a composition capable of enabling the formulation of cannabidiol for topical applications and capable of effectively delivering cannabidiol through the skin.

### SUMMARY OF THE INVENTION

Applicant has faced the problem of topical administration of cannabidiol and has surprisingly found a formulation of cannabidiol incorporated into niosomes capable of overcoming the drawbacks known in the art.

In particular, the Applicant has faced the problem of making a topical composition comprising cannabidiol in the form of an aqueous-based emulsion that is stable over time and does not give rise to separation and/or precipitation and/or alteration of its components.

At the same time, the Applicant faced the issue of making a topical composition comprising cannabidiol capable of solubilizing a greater amount of cannabidiol.

Finally, the Applicant faced the problem of making a topical composition comprising cannabidiol that would improve the penetration of cannabidiol through the skin.

After extensive experimentation, Applicant found that a topical composition comprising a formulation of cannabidiol incorporated into niosomes comprising at least one linear or branched polyglycerol, esterified with saturated or monounsaturated linear fatty acids, (ii) at least one polysaccharide selected from the group consisting of pullulan, glucan, alginate, amylose, glycogen, and inulin, preferably a beta-glucan, and optionally, (iii) at least one glycol having 4 to 16 carbon atoms, preferably caprylyl glycol, enables up to 5% or more by weight of cannabidiol to be solubilized and enhances the penetration of cannabidiol through the skin, thereby making more cannabidiol available and effective for the same amount of product administered.

In particular, the Applicant found that the composition of the invention exhibited increased elasticity, estimated through extrusion measurements, aided by the presence of beta-glucan which gives the niosomes improved plasticity and deformability in crossing the intercellular spaces of the skin.

The Applicant further found that the resulting topical composition was stable over time, did not cause any irritation, and had adequate anti-microbial resistance.

Finally, the Applicant found that the topical composition thus obtained, in addition to providing effective protection from atmospheric pollutants, such as particulate matter, was also capable of improving skin cell viability and cell trophism, and of exerting a significant anti-inflammatory ability, partly related to the improved penetration of the cannabidiol component, and partly related to the presence of beta-glucan.

Moreover, the Applicant also found that the composition of the invention has considerable soothing and anti-itch activity in the skin, together with stimulation of mitogenesis in hair bulb cells. On the other hand, preliminary evaluations, still in progress, suggest that the composition of the invention has considerable pain-relieving activity in the skin.

The Applicant also believes that the results obtained with cannabidiol, in terms of stability and penetration ability, can be extended to other cannabinoids, such as, for example, the aforementioned cannabigerol (CBG) and cannabinol (CBN).

Therefore, a first object of the present invention relates to a topical composition comprising an aqueous-based composition comprising cannabinoids, in particular cannabidiol, in niosomes having size lower than 500 nm, and at least one topically acceptable excipient, characterized in that said niosomes comprise (i) at least one linear or branched polyglycerol, esterified with saturated or monounsaturated linear fatty acids, (ii) at least one polysaccharide selected from the group consisting of pullulan, glucan, alginate, amylose, glycogen, and inulin, and optionally (iii) at least one glycol having 4 to 16 carbon atoms.

A second aspect of the present invention relates to an aqueous-based composition, in particular an aqueous dispersion or solution, comprising cannabinoids, in particular cannabidiol, incorporated in niosomes, characterized in that said niosomes comprise (i) at least one linear or branched polyglycerol esterified with saturated or monounsaturated linear fatty acids, (ii) at least one polysaccharide selected from the group consisting of pullulan, glucan, alginate, amylose, glycogen, and inulin, and optionally (iii) at least one glycol having 4 to 16 carbon atoms.

A third aspect of the present invention relates to a method for preparing an aqueous-based composition comprising niosomes containing cannabinoids, in particular cannabidiol, comprising the use of hand shaking or ultrasonic shaking techniques, characterized in that said niosomes comprise (i) at least one linear or branched polyglycerol, esterified with saturated or monounsaturated linear fatty acids, (ii) at least one polysaccharide selected from the group consisting of pullulan, glucan, alginate, amylose, glycogen, and inulin, and optionally (iii) at least one glycol having 4 to 16 carbon atoms.

**BRIEF DESCRIPTION OF THE FIGURES**

The present invention will be best illustrated in the following detailed description, set forth below with reference to the accompanying drawings, which are provided for illustrative purposes only and, therefore, are not limiting, in which:

Figure 1 shows a photomicrograph of the niosome dispersion vesicles (CBD-S5) of Example 1 observed by TEM transmission electron microscopy (JEM-1200EX, JEOL Co., Tokyo, Japan) using a negative staining method,

Figure 2 shows the graph of the Dynamic Light Scattering (DLS) obtained by analyzing the same sample of the niosome dispersion vesicles (CBD-S5) as in Example 1 with a Brookhaven 90Plus-Particle Size Analyzer instrument,

Figure 3 shows the graph of the relative cell viability of a reconstructed human epidermis model treated with niosome dispersion (CBD-S5) of Example 1 against a positive control (PC) and a negative control (NC) as described in Example 5A,

Figure 4 shows the graph of the relative cell viability of a reconstructed human epidermis model treated with the niosome dispersion (CBD-S5) of Example 1, in the presence or absence of PM2.5, against a positive control (PC) and a negative control (NC) as described in Example 8, and

Figure 5 shows the graph of the relative percent IL-1$\alpha$ release of a reconstructed human epidermis model treated with niosome dispersion (CBD-S5) of Example 1, in the presence or absence of PM2.5, against a positive control (PC) and a negative control (NC) as described in Example 8.

## DETAILED DESCRIPTION OF THE INVENTION

The present invention relates in a first aspect to a topical composition comprising an aqueous-based composition comprising cannabinoids, in particular cannabidiol, in niosomes having size lower than 500 nm, and at least one topically acceptable excipient, characterized in that said niosomes comprise (i) at least one linear or branched polyglycerol esterified with saturated or monounsaturated linear fatty acids, (ii) at least one polysaccharide selected from the group consisting of pullulan, glucan, alginate, amylose, glycogen, and inulin, and optionally (iii) at least one glycol having 4 to 16 carbon atoms. The expression "topically acceptable" as used herein is intended to define substances recognized as having no adverse side effects (irritation, toxicity, and so forth) when applied to the skin, epithelia and/or mucous membranes.

The expression "niosome" or "niosomes" as used herein is intended to define hydrophilic vesicles formed from non-ionic surfactant oriented in a bilayer.

Advantageously, the niosomes used in the present invention have diameters of less than 400 nm, more preferably less than 300 nm, and even more preferably less than 200 nm. Preferably, the niosomes used in the present invention have diameters greater than 10 nm, more preferably greater than 20 nm, and even more preferably greater than 40 nm. Advantageously, the niosomes used in the present invention have diameters between 50 nm and 180 nm, preferably between 70 nm and 150 nm.

The linear or branched polyglycerol esterified with saturated or monounsaturated linear fatty acids useful in the present invention is obtained by esterification of a linear or branched polyglycerol with saturated or monounsaturated linear fatty acids.

The cannabidiol useful in the present invention may be of natural and/or synthetic origin, preferably having a purity equal to or higher than 95%, equal to or higher than 96%, equal to or higher than 97%, equal to or higher than 98%, or equal to or higher than 99. Advantageously, the cannabidiol useful in the present invention has a purity of about 100%.

Naturally derived cannabidiol may contain minority percentages of other cannabinoids, such as, for example, cannabichromene, cannabigerol, and cannabinol, generally in the aggregate equal to or lower than 5%, equal to or lower than 4%, equal to or lower than 3%, equal to or lower than 2%, or equal to or lower than 1%.

Preferred examples of polyglycerols are triglycerol, tetraglycerol, hexaglycerol, octaglycerol, decaglycerol. Linear or branched polyglycerols useful in the present invention are commercially available.

Examples of commercial products are polyglycerols distributed by American International Chemical LLC under the trade name Polyglycerol-3, by Spiga Nord S.p.A. under the trade names Vegetable Polyglycerine-3, Vegetable Polyglycerine-4, Vegetable Polyglycerine-6, and Vegetable Polyglycerine-10, and by Solvay Chemicals, Inc. under the trade names Polyglycerol-3 and Polyglycerol-4.

Useful examples of saturated linear fatty acids include monocarboxylic acids having 4 to 32 carbon atoms, such as butyric acid, valeric acid, caproic acid, enanthic acid, caprylic acid, pelargonic acid, capric acid, lauric acid, myristic acid, palmitic acid, margaric acid, stearic acid, arachidic acid, behenic acid, lignoceric acid, cerotic acid, montanic acid, melissic acid, and lacceroic acid.

Preferred saturated linear fatty acids include monocarboxylic acids having 12 to 22 carbon atoms, such as lauric acid, myristic acid, palmitic acid, margaric acid, stearic acid, arachidic acid, and behenic acid.

Useful examples of linear monounsaturated fatty acids include monocarboxylic acids having 14 to 24 carbon atoms, such as myristoleic acid, palmitoleic acid, oleic acid, gadoleic acid, and erucic acid.

Useful examples of mixtures of fatty acids are vegetable oils obtained by pressing or extracting seeds or fruits, such as, for example, olive oil, peanut oil, coconut oil, palm oil, and rapeseed oil. Due to the low content of polyunsaturated acids, olive oil and coconut oil are preferred, particularly olive oil.

Esters of linear or branched polyglycerols with linear saturated or monounsaturated fatty acids, or mixtures thereof, useful in the present invention are commercially available. Examples of commercial products are polyglycerol esters made and distributed by the company Lonza under the trade name Polyaldo, such as, for example, Polyaldo® 6-2-S [Polyglyceryl-6 Disterate], Polyaldo®10-1-S [Polyglyceryl-10 Stearate], Polyaldo® 10-1-O [Polyglyceryl-10 Oleate], Polyaldo® 10-2-P [Polyglyceryl-10 Dipalmitate], by the company Hydrior AG, Germany under the trade name Hydriol, such as, for example, HYDRIOL® PGO (Polyglycerol-4-oleate), HYDRIOL® PGD (Polyglycerol-3-diisostearate), by the company Naturalis s.r.l. under the trade name Soavirol, such as, for example, Soavirol OV6 (olive oil polyglyceryl-6 ester), Soavirol OV4 (olive oil polyglyceryl-4 ester), and by the company Nikko Chemicals Co, Ltd. under the trade name Nikkol Hexaglyn, such as, for example, Nikkol Hexaglyn 1-L (polyglyceryl-6 laurate) and Nikkol Hexaglyn PR-15 (polyglyceryl-6 polyricinoleate). Additional suppliers of polyglycerol esters for cosmetic applications can be found at https://cosmetics.specialchem.com/selectors?q=polyglyceryl.

The polysaccharide useful in the present invention is selected from the group consisting of polysaccharides of natural origin, such as pullulans, glucans alginates, amylose, glycogen, and inulin.

Advantageously, the polysaccharide useful in the present invention is selected from the group consisting of alpha- and beta-glucans, more preferably beta-glucans.

Beta-glucans are linear polysaccharides comprising glucose molecules joined together by $\beta(1-3)$ glycosidic bonds. Beta-glucans are natural products found in cereals, bacteria and fungi. Oats and barley are particularly rich in beta-glucans, and their production comes mainly from extraction from these cereals. Beta-glucans obtained from fungi are also widely available commercially. Beta-glucans from fungi and yeasts contain branched bonds with glycosidic and $\beta(1-6)$ bonds, while beta-glucans from cereals have glycosidic and $\beta(1-3)$ and $\beta(1-4)$ bonds. Beta-glucans derived from cereals are more soluble in water, and are therefore preferred for the purposes of the present invention.

Beta-glucans particularly useful for the purposes of the present invention are beta-glucans distributed by the company Ohly GmbH, Germany, under the trade name Ohly-GOO Glucan, by the company Maypro Industries LLC under the trade name Chitoglucan®, by the company Lesaffre Human Care under the trade name Lynside® Wall Glucan, and by the company HerbaKraft Inc. under the trade name Beta Glucan. Additional suppliers of beta-glucans for cosmetic applications can be found at https://cosmetics.specialchem.com/inci/beta-glucan.

The glycol having 4 to 16 carbon atoms useful in the present invention is preferably selected from the group consisting of 1,2-butanediol, 1,2-pentanediol, 1,2-hexanediol, 1,2-heptanediol, 1,2-octanediol (caprilyl glycol), 1,2-decanediol (capril glycol), 1,2-dodecanediol (lauryl glycol), and 1,2-hexadecanediol.

1,2-hexanediol, 1,2-heptanediol, 1,2-octanediol (caprilyl glycol), 1,2-decanediol (capril glycol) are particularly preferred. For the purposes of the present invention, 1,2-octanediol (caprylyl glycol) is advantageously used.

Commercial products comprising glycols useful for the purposes of the present invention are distributed by the company CBW Chemie GmbH, Germany, by the company Inolex Inc., USA under the trade name Lexgard®, by the company Wintersun Chemical, USA, and by the company Chemos GmbH & Co. KG.

Suppliers of polyglycerols and their esters, beta-glucans and glycols useful in the present invention can also be found on the Internet, for example at https://www.food-ingredients.com/english/, at http://www.buyersguidechem.com/ and at https://cosmetics.specialchem.com/.

In manufacturing the niosomes employed for the purposes of the present invention, further components suitable for stabilizing and preserving the aqueous solution/dispersion of niosomes are preferably used, such as for example water-soluble natural antioxidants such as ascorbic acid and its derivatives, and polyphenols.

The aqueous-based composition comprising niosomes of the present invention can be prepared according to techniques known in the art, for example, by the hand shaking technique or by the ultrasonic technique.

The hand shaking technique comprises a first step of solubilization of the components in a volatile organic solvent, such as, for example, ethyl ether, chloroform or methanol, conducted in a glass flask, a second step of evaporation, conducted in a rotary evaporator at room temperature (20°-25°C) leaving a thin layer of the components deposited on the walls of the flask, and finally a third step of rehydration with an aqueous phase comprising the plant extracts at a temperature between 0° and 60°C under slight agitation.

The ultrasound technique comprises the sonication at a temperature between 0° and 60°C of a dispersion obtained by mixing an organic phase comprising surfactants and an aqueous phase comprising plant extracts.

These and other methods of preparing compositions comprising niosomes are described in the literature, for example in the article by Madhav et al., "Niosomes: a novel drug delivery system"; International journal of research in pharmacy and chemistry, IJRPC 2011, 1(3), 498-5.11.

The niosome dispersion/solution of the present invention preferably comprises the amounts of components described below and expressed as a weight percentage with respect to the total weight of the niosome dispersion/solution (% w/w).

Cannabidiol is included in the niosome dispersion/solution up to an amount of 10% w/w. Preferably, the cannabidiol is present in an amount ranging from 1% to 8% w/w, more preferably ranging from 2% to 7% w/w, and even more preferably ranging from 3% to 6% w/w. Advantageously, the amount of cannabidiol ranges from 4% to 5% w/w.

The dispersion/niosome solution comprises an amount of polyglycerol esters ranging from 40% to 90% w/w, preferably ranging from 50% to 80% w/w.

The amount of beta-glucan included in the niosome dispersion/solution ranges from 1% to 5% w/w, preferably from 1% to 3% w/w.

The amount of glycol included in the dispersion / solution of niosomes ranges from 0.1% to 5% w/w, preferably from 0.5% to 3% w/w.

The resulting niosome dispersion/solution comprises water in an amount of from 10% to 40% w/w, preferably from 20% to 30% w/w.

The niosome dispersion/solution may comprise other components, such as for example stabilisers and preservatives, up to the amount of 1% w/w.

The topical composition of the present invention may be liquid or semi-solid.

**EP 4 114 355 B1**

In particular, the topical composition of the present invention is a cosmetic composition and/or a medical device and/or a pharmaceutical preparation, for application to the skin, epithelia and mucous membranes.

The topical composition of the present invention advantageously comprises liquid or semi-solid compositions within which the aqueous-based niosome composition is dispersed in an amount of from 0.5% to 10% by weight, preferably from 1% to 5% by weight, relative to the total weight of the topical composition.

The liquid compositions of the present invention include solutions, emulsions, microemulsions, lotions, gels, foams, milks, micellar waters, oils, tensiolites or suspensions with a wide variation in viscosity.

Liquid compositions include, for example, aqueous solutions, hydro-alcoholic solutions, oily solutions, emulsions obtained by dispersion of an oily phase in an aqueous phase (oil-in-water) or, vice versa, of an aqueous phase in an oily phase (water-in-oil), and suspensions, obtained by dispersion of a dispersed phase, consisting of solid particles, in a dispersing medium generally represented by an aqueous or oily liquid of a certain viscosity.

The semi-solid compositions of the present invention include creams, gels, balms, ointments, pastes, cream-gel, sticks and waxes.

In addition, the topical compositions of the present invention may comprise various topically acceptable additives or vehicles useful in the preparation of cosmetic products and/or medical devices and/or pharmaceutical preparations known to the man skilled in the art, such as, for example, emulsifiers, moisturizers, solvents, emollients, stabilisers, viscosifiers, preservatives, lubricants, sequestering or chelating agents, fillers, powders, fragrances, perfumes, absorbents, colorants and opacifiers, antioxidants, vitamins, natural extracts, polysaccharides, shielding substances, UV filters, essential oils, keratin-active substances, and amino acids.

Suitable solvent additives include, for example, water, alcohols, ketones (such as acetone and methyl isobutyl ketone), glycols (such as ethylene glycol, propylene glycol and butylene glycol), polyethylene glycols (such as PEG-40, PEG-50, PEG-60), alkyl acetates (such as amyl acetate, isopropyl acetate, butyl acetate), paraffins and isoparaffins, cycloalkyls (such as cyclohexane), glycerin, natural and synthetic oils, natural and synthetic triglycerides, essential oils.

Advantageously, the topical compositions of the present invention are aqueous compositions.

In the aqueous compositions, water represents the main component of the composition reaching even an amount of up to 99% by weight with respect to the weight of the total composition. The aqueous compositions preferably contain an amount of water ranging from 25% to 99%, preferably ranging from 35% to 95%, and more preferably ranging from 50% to 90% by weight relative to the weight of the total composition.

The aqueous compositions of the present invention may preferably comprise a total amount of non-aqueous solvents ranging from about 0.1% to about 60%, more preferably ranging from 1% to 40%, and even more preferably ranging from 5% to 35% by weight relative to the weight of the total composition.

Examples of suitable emulsifying additives are non-ionic, cationic, anionic and amphoteric surfactants, or a combination thereof. Examples of useful emulsifiers in the present invention are sorbitans, ethoxylated long-chain alcohols, alkyl polyglycosides, soaps, alkyl sulfates, such as, for example, sodium cetylstearyl sulfate, monoalkyl and dialkyl phosphates, alkyl sulfonates, hydrogenated castor oil, acyl isothionates, sucrose esters, betaines, lecithins, quaternary ammonium salts, alkyloleates, glycerides, such as, for example, caprylocaproyl polyoxylglycerides and emulsifiers from olive oil.

Preferably, the composition of the present invention comprises a total amount of emulsifiers ranging from about 0.1% to about 60%, more preferably ranging from 0.5% to 25%, and even more preferably ranging from 0.5% to 10% by weight relative to the weight of the total composition.

Typical viscosity additives useful in the present invention are, for example, xanthan gum, hydroxypropylcellulose, hydroxyethylcellulose, carbopol, carrageenans, polyoxamers, and acacia gum.

Advantageously, the composition of the present invention comprises a total amount of viscosifiers ranging from about 0.1% to about 25%, more preferably ranging from 0.5% to 10%, and even more preferably ranging from 0.5% to 5% by weight relative to the weight of the total composition.

Examples of additives with moisturizing action useful in the present invention are, for example, urea, allantoin, hyaluronic acid and derivatives thereof, glycerin, amino acids, acetylmonoethanolamide, butoxypropanol, butyl glycol, low molecular weight polyethylene glycols (such as PEG-40, PEG-50, PEG-60), aloe, mallow, trehalose and sorbitol.

Preferably, the composition of the present invention comprises a total amount of moisturizers ranging from about 0.05% to about 25%, more preferably ranging from 0.5% to 10%, and even more preferably ranging from 0.1% to 5% by weight of the total composition.

Examples of suitable emollient additives useful in the present invention include, for example, lanolin, almond oil, olive oil, vegetable oils, jojoba oil, argan oil, hydrogenated castor oil, natural lipophilic extracts, microcrystalline wax, polydimethylsiloxane (dimethicone), polymethylphenylsiloxane, glycol and silicone polymers, mineral oils, paraffin, ozokerite, ceresin, triglyceride esters, acetylated monoglycerides, ethoxylated glycerides, alkyl esters of fatty acids, fatty acids, long chain alcohols, sterols, beeswax, polyhydric alcohols, polyesters, and fatty acid amides.

Preferably, the composition of the present invention comprises a total amount of emollients ranging from about 0.1% to about 25%, more preferably ranging from 0.5% to 10%, and even more preferably ranging from 0.5% to 5% by weight relative to the weight of the total composition.

Examples of useful fragrances in the present invention are, for example, natural essential oils or fractions or concentrates of essential oils, such as, for example, lemon oil, bergamot oil, lavender oil, limonene, linalool. Preferably, the composition of the present invention comprises a total amount of fragrances ranging from about 0.001% to about 0.1%.

Examples of suitable preservative additives useful in the present invention include, for example, alcohols, such as ethanol, phenoxyethanol and benzyl alcohol, methyl and propyl parahydroxybenzoate, butylated hydroxyanisole (BHA), sorbates, urea derivatives, and isothiazolinones, natural preservatives, such as, for example, ascorbic acid and derivatives, tocopherol and derivatives, polyphenols. Further examples of dyes that can be used in the topical composition of the present invention can be found in Annex V to Regulation (EC) No 1223/2009 of 30 November 2009.

Preferably, the composition of the present invention comprises a total amount of preservatives ranging from about 0.01% to about 2.00%, more preferably ranging from 0.05% to 1.00%, and even more preferably ranging from 0.1% to 0.5% by weight relative to the weight of the total composition.

Examples of sequestrant or chelating additives useful in the present invention are EDTA, HEDTA, alkyl oxalates, lithium or potassium oxalate, sodium or potassium pyrophosphate. Preferably, the composition of the present invention comprises a total amount of sequestering or chelating additives ranging from about 0.01% to about 20%, more preferably ranging from 0.05% to 10%, and even more preferably ranging from 0.1% to 5% by weight relative to the weight of the total composition.

Examples of suitable stabilizing additives useful in the present invention are long chain alcohols (such as cetyl alcohol, stearyl alcohol) and mixtures thereof, high molecular weight polyethylene glycols (such as PEG-9000 and PEG 14000) and polyvinylpyrrolidones (such as povidone).

Preferably, the composition of the present invention comprises a total amount of stabilizers ranging from about 0.1% to about 25%, more preferably ranging from 0.5% to 15%, and even more preferably ranging from 1% to 10% by weight relative to the weight of the total composition.

Examples of suitable powder additives useful in the present invention are elastomeric silicones such as dimethicone/vinyldimethicone crosspolymers (DC 9506, Dow-Corning), mixtures of cyclomethicone and dimethicone crosspolymers (DC 9040, Dow Corning), silica-treated crosspolymers of dimethicone and vinyl dimethicone (DC 9701, Dow Corning), mixtures of crosspolymers of cyclomethicone and dimethicone/vinyldimethicone (SFE 839, GE Bayer Silicones).

Preferably, the composition of the present invention comprises a total amount of powder additives ranging from about 0.1% to about 5%, more preferably ranging from 0.2% to 1%, by weight relative to the weight of the total composition.

Examples of opacifying agents useful in the present invention are zinc or aluminum oxide, titanium or zinc dioxide, alumina, mica, fatty acid salts with aluminum, and gypsum.

Examples of dyes preferably used in the present invention are easily washable water-soluble dyes that do not stain the skin and do not leave residue such as, for example, Acid Blue 3 C.1.42051, Acid Blue 9 C.I.42090, Acid Blue 74 C.1.73015, Pigment Blue 15 C.1.74160, Acid Yellow 3 C.1.47005, Food Yellow 3 C.1.15985, Acid Yellow 23 C.1.19140, Acid Yellow 73 C.1.45350, Acid Red 14 C.1.14720, Acid Red 18 C.1.16255, Acid Red 27 C.1.16185, Acid Red 51 C.I.45430, Acid Green 1 C.1.10020, Acid Green 25 C. 1.61570, and mixtures thereof. Further examples of dyes that can be used in the topical composition of the present invention can be found in Annex IV of Regulation (EC) No 1223/2009 of 30 November 2009.

Preferably, the composition of the present invention comprises a total amount of opacifying agents and colorants ranging from about 0.01% to about 15%, more preferably ranging from 0.05% to 5% by weight relative to the weight of the total composition.

Preferably, the composition of the present invention may comprise UV filters capable of shielding the skin from the action of ultraviolet radiation. Examples of UV filters are, for example, acrylates such as 2-ethylhexyl 2-cyano-3,3-diphenylacrylate (PARSOL 340) and ethyl 2-cyano-3,3-diphenylacrylate, camphor derivatives such as camphor 4-methyl benzylidene (PARSOL 5000) and camphor 3-benzylidene, cinnamates such as octyl methoxycinnamate (PARSOL MCX), ethoxyethyl methoxycinnamate, diethanolamine methoxycinnamate (PARSOL Hydro), triazone derivatives such as ethylhexyl triazone (UVINUL T-150), diethylhexyl butamido triazone (UVASORB HEB), dibenzoylmethane derivatives such as 4-tert-butyl-4'-methoxydibenzoylmethane (PARSOL 1789), dimethoxydibenzoylmethane, benzotriazole derivatives such as 2,2'-methylene-bis-(6-(2H-benzotriazol-2-i1)-4-(1,1,3,-tetramethylbutyl)-phenol (TINOSORB M), triazine derivatives such as bis-ethylhexyloxyphenol methoxyphenyl triazine (TINOSORB S). Other examples of UV filters that can be used in the topical composition of the present invention can be found in Annex VI to Regulation (EC) No 1223/2009 of 30 November 2009.

Preferably, the composition of the present invention comprises a total amount of UV filters ranging from about 0.1% to about 20%, more preferably ranging from 0.5% to 15% by weight relative to the weight of the total composition.

The following experimental part illustrates at least one embodiment of the invention, without however in any way restricting the scope of protection as defined in the claims appended to the present description.

**EXPERIMENTAL PART**

**EXAMPLE 1** - **Preparation of niosomes**

A dispersion of niosomes (CBD-S5) comprising cannabidiol (CBD) was prepared with the composition shown in the following Table 1. The resulting composition had the appearance of a uniform pale yellow viscous gel. The weight percentage relative to the weight of the total composition is expressed for each component.

TABLE 1

| Component | CBD-S5 |
|---|---|
| Cannabidiol | 5 |
| Polyglyceryl-6 oleate | 30 |
| Polyglyceryl-10 caprate | 20 |
| Polyglyceryl-4 caprate | 20 |
| Beta-glucan | 2 |
| Capryloyl glycol | 1 |
| Water | q.b. to 100 |

**EXAMPLE 2**

The vesicle morphology of the niosome dispersion (CBD-S5) of Example 1 was examined by TEM transmission electron microscopy (JEM-1200EX, JEOL Co., Tokyo, Japan) using a negative staining method.

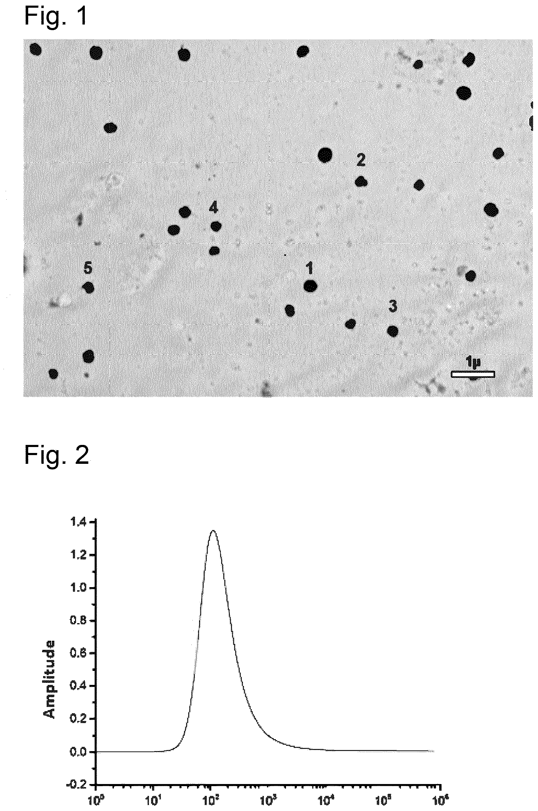

A small aliquot (5.0 ml) of the CBD-S5 dispersion was centrifuged at 4°C for 30 min at a rate capable of developing approximately 80000xg. The pellet was dried and then diluted with 1% uranyl acetate solution. A drop of the suspension was directly used to load a carbon-coated electron microscope screen. Excess sample was removed by absorption using plain filter paper. The screen thus prepared was incubated at 30°C for 10 minutes to dry thoroughly and observed by TEM at 80 kV. A photomicrograph of the observed vesicles is shown in Figure 1. The numbered vesicles had the diameter as shown in the following Table 2.

TABLE 2

| Vesicle No. | Diameter |
|---|---|
| 1 | 321 ± 16 |
| 2 | 198 ± 10 |
| 3 | 191 ± 9 |
| 4 | 188 ± 9 |
| 5 | 185 ± 8 |

Ultrastructural analysis by transmission electron microscopy showed the presence of spheroidal vesicular structures with an average size of about 200 nm.

Figure 2 shows the Dynamic Light Scattering (DLS) graph obtained by analyzing the same sample of CBD-S5 with a Brookhaven 90Plus-Particle Size Analyzer. The measurement confirmed an average diameter of 190.4 nm with a poly-dispersability index of 0.137.

**EXAMPLE 3**

Elasticity is one of the most important characteristics of niosomes. Elasticity is estimated by extrusion measurements. The vesicles of the niosome dispersion (CBD-S5) of Example 1 were extruded through a polycarbonate filter with pore diameter of 100 nm under constant pressure. Elasticity is expressed in terms of the deformability index (D) calculated using the following formula:

$$D = J \times (rv / rp)2$$

where J is the ratio of suspension extruded in 5 minutes, rv is the size of the vesicles after crossing the filter as measured by DLS, and rp is the pore diameter of the filter.

Good deformability is demonstrated when the deformability index is greater than 80. The test was conducted with the Avestin LiposoFast instrument by measuring the particles with the Brookhaven 90Plus-Particle Size Analyzer.

The results showed a 5-minute extruded suspension ratio (J) of 87% with rv vesicle size of 141 nm. The deformability index (D) was therefore 172.96, confirming the excellent deformability of the niosomes, a characteristic predictive of an excellent ability to penetrate through the skin, epithelia and skin appendages. Characteristics of elasticity and deformability are essential for interaction with the superficial layers of the skin and skin appendages, promoting absorption and penetration not only through chemical interactions.

**EXAMPLE 4**

The niosome dispersion vesicles (CBD-S5) from Example 1 and a 5% solution of cannabidiol in MCT (CBD-5%) were evaluated for their ability to diffuse through an artificial membrane, using phosphate buffered saline (PBS) as a negative control. The medium-chain triglyceride oils (MCTs) used for comparison are among the most popular cannabidiol solubilization systems even for topical use.

The assay included the evaluation of cannabidiol diffusion with Franz cells through a Strat-M® artificial membrane (Sigma-Aldrich) used for trans-membrane diffusion tests, which simulate the diffusion in the human epidermis of different types of compounds and formulations.

The artificial Strat-M® membrane consists of two layers of polyether sulfone (PES, more diffusion-resistant) placed on the surface and a layer of polyolefin (more diffusive). These polymer layers create a porous structure with a diffusion gradient. In addition, this porous structure is impregnated with a mixture of synthetic lipids, giving it skin-like properties.

The diffusion cell consists of a donor chamber and a receptor chamber between which the membrane is placed. In the receptor chamber there is phosphate buffer with 5% bovine serum albumin (BSA) and a magnet for adequate mixing of the solution. The area available for sample application is 0.2 cm$^2$.

The CBD-S5 and CBD-5% samples were applied to the membrane (1g/cm$^2$) using a special Gilson P100 positive displacement pipette for dense liquids. The negative control was membranes treated with phosphate buffered saline (PBS) only.

Samples and controls were incubated at 32°C, 5% $CO_2$ and exposure was carried out for 16, 24 and 48 hours. The sample was tested in triplicate. At the different exposure times, some of the receptor fluid was withdrawn with a syringe. The samples thus recovered were immediately frozen at -20°C and thawed subsequently for quantitative chromatographic analysis.

An aliquot of the amount diffused under the membranes at each evaluation time was diluted with a methanol solution acidified with 0.3% phosphoric acid (v/v). The subnatant was analyzed by HPLC-DAD with the WATERS 2695 instrument, with the following analytical conditions.

Wavelength : 212 nm

Injection : 10 $\mu$l

Column : Eclipse XDB-C18 3.5$\mu$m 3,0x150mm

Column temperature : 35°C

Flow rate : 0,6 ml/min

Mobile phase A : 0.3% $H_3PO_4$ in $H_2O$ milliQ

Mobile phase B : 0,3% $H_3PO_4$ in ACN

Gradient: As per Table 3

TABLE 3

| Minutes | Phase A | Phase B |
|---------|---------|---------|
| 0 | 45 | 55 |
| 5 | 45 | 55 |
| 21 | 25 | 75 |
| 21.1 | 0 | 100 |
| 25 | 0 | 100 |

(continued)

| Minutes | Phase A | Phase B |
|---------|---------|---------|
| 25.1 | 45 | 55 |
| 35 | 45 | 55 |

The results are summarized in the following Table 4.

TABLE 4

| Sample | Permeation rate at different exposure times ($\mu$g/cm$^2$) | | | | | |
|--------|----------|---------|----------|---------|----------|---------|
| | 16 hours | Average | 24 hours | Average | 48 hours | Average |
| CBD-S5 | 22.75 | | 22.22 | | 74.79 | |
| | 26.22 | 23.66 | 35.86 | 28.71 | 87.04 | 74.49 |
| | 22.00 | | 28.06 | | 61.66 | |
| CBD-5% | 20.93 | | 20.49 | | 29.14 | |
| | 18.14 | 19.28 | 17.80 | 19.72 | 26.71 | 28.11 |
| | 18.77 | | 20.87 | | 28.49 | |

The results of Table 4 demonstrated a significant increase in cannabidiol diffusion of the CBD-S5 sample of the present invention compared to the CBD-5% sample.

The results of the test allow for the prediction of an increased ability to penetrate and release active ingredient into the skin.

**EXAMPLE 5**

The irritating power of the niosome dispersion (CBD-S5) of example 1 was assessed by an in vitro test on reconstituted epidermis and an in vivo test on a group of twenty subjects. Determining whether topical and cosmetic products are irritants is an important assessment for establishing procedures for correct and safe use.

*A. In vitro test*

The in vitro test consists of a topical exposure of the test substance on reconstituted human epidermis (RHE) followed by a cell viability test.

Cell viability is determined by the conversion of MTT (3-(4,5-dimethylthiazol-2-yl)-2,5-diphenyltetrazolium bromide) to a formazan blue salt, which is quantitatively measured after extraction from tissue. Conversion occurs via the mitochondrial enzyme succinate dehydrogenase, which is only active in living cells, causing the MTT tetrazole ring to open and formazan to form. Assessment of the viability of tissues exposed to the test substance compared to the negative control (treated with Dulbecco's phosphate buffered saline - DPBS) and the positive control (treated with sodium dodecyl sulphate - SDS) is used to predict skin irritation potential.

The reconstructed human epidermis model consists of normal human-derived epidermal keratinocytes cultured to recreate a highly differentiated multilayered human epidermis. It consists of a basal layer, a spinous and granular layer and a multilayered stratum corneum containing intercellular lamellar lipid layers arranged in a manner similar to those present in vivo.

The reconstructed skin sample covers an area of 0.63 cm$^2$. The test is performed in triplicate. Each sample is treated with 25 $\mu$g CBD-S5. In parallel, the positive control (PC) test is performed by applying 30 $\mu$L of 5% SDS solution to the skin sample and the negative control (NC) test by applying 30 $\mu$L of DPBS to the skin sample. The treatment takes 60 minutes, then the samples are washed to remove the test substances and incubated for 24 hours before performing the MTT assay.

The MTT assay is performed by transferring the samples to 24-well plates containing a solution of MTT (1 mg/ml). The blue formazan salt produced by the cellular mitochondria is extracted with 2.0 ml isopropanol per sample.

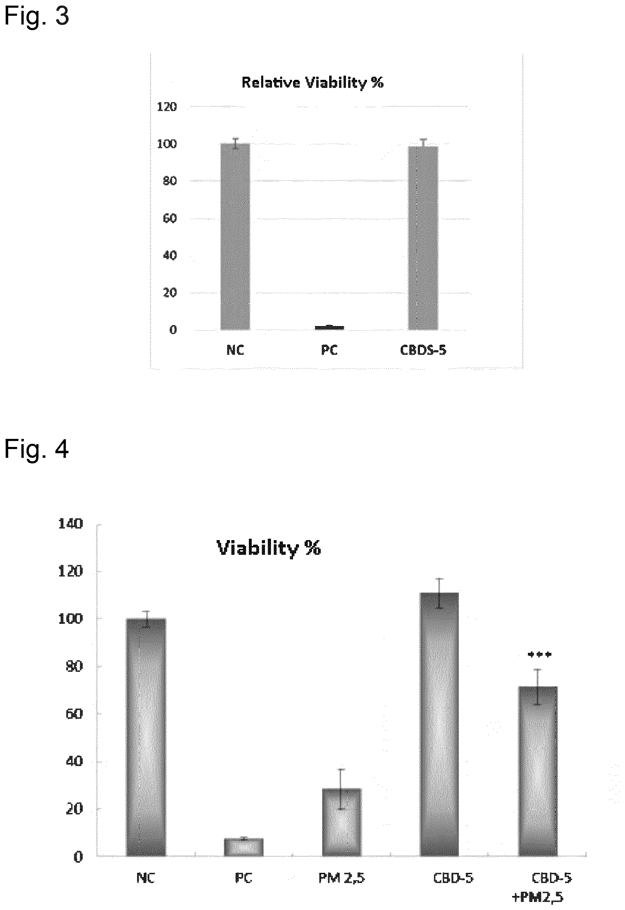

The optical density of the extracted formazan is determined spectrophotometrically at a wavelength of 570 nm using the $\mu$QUANT BIO TEK Microplate Spectrophotometer. Relative cell viability is calculated for each tissue as % of the mean optical density of the negative control tissues. The results are summarized in the following Table 5 and in Figure 3.

TABLE 5

| Sample | Mean relative viability (%) | Standard Deviation |
|---|---|---|
| NC | 100.00 | 2.9 |
| PC | 2.16 | 0.5 |
| CBD-S5 | 98.70 | 3.5 |

The dispersion of niosomes (CBD-S5) from Example 1 was therefore found to be nonirritating.

*B. In vivo test*

The in vivo test consisted of applying the niosome dispersion (CBD-S5) from Example 1 using Finn Chambers (20 microlitre volume cells) to the back and/or forearm of selected subjects. The irritant potential of the product was assessed by testing at three separate times. Immediate skin irritation potential was assessed by applying the product for 60 minutes (T1). The skin irritant potential was assessed by applying the product for 48 hours (T2) and 24 hours after removal of the product (T3). The assessment was divided into four levels of irritation as described in the following Table 6.

TABLE 6

| +/- | doubtful reaction (mild non-uniform erythema) |
|---|---|
| + | mild uniform red-red erythema |
| ++ | very obvious bright red erythema, moderate degree, sharp borders, possible non-uniform oedema |
| +++ | severe erythema with diffuse oedema, possible vesicles and/or pustules at follicular level |

The following Table 7 summarizes the results obtained on the subjects investigated at the end of the test.

TABLE 7

| | Irritated subjects at time | | |
|---|---|---|---|
| | T1 | T2 | T3 |
| +/- | 0 | 0 | 0 |
| + | 0 | 0 | 0 |
| ++ | 0 | 0 | 0 |
| +++ | 0 | 0 | 0 |

The in vivo test confirmed the non-irritability of the niosome dispersion (CBD-S5) of Example 1.

**EXAMPLE 6** - **Anti-microbial resistance test (challenge test)**

The test consists of "challenging" the preparation with a specified inoculum of suitable micro-organisms, leaving the inoculated preparation at a prescribed temperature and withdrawing samples from the container at specified time intervals and counting the number of organisms present in the samples taken.

The test was conducted following the recommendations in the Italian Pharmacopoeia - Edition IX and equivalent European Pharmacopeia 5.1.3. and using bacterial cultures of the following strains of microorganisms.

- Pseudomonas aeruginosa ATCC 9027
- Escherichia coli ATCC 8739
- Staphylococcus aureus ATCC 6538
- Candida albicans ATCC 10231

- Aspergillus brasiliensis ATCC 16404

The niosome dispersion (CBD-S5) of Example 1 was inoculated with the different microorganisms with an inoculum comprising a number of bacteria between $10^5$ and $10^6$ and a number of fungi or yeasts between $10^4$ and $10^5$. The inoculated product was stored at room temperature (20°-25°C) away from light. A sample of the product was taken at time t0 (basal value) and at 2 (t2), 7 (t7), 14 (t14), and 28 (t28) days intervals, for determination of the number of micro-organisms present.

The results are summarized in the following Table 8, where the values found, expressed on a logarithmic basis, for each micro-organism at the various observation times are shown. The data express the colony-forming units (cfu) relative to 1 g of product.

TABLE 8

|  | $t_0$ | $t_2$ | $t_7$ | $t_{14}$ | $t_{28}$ |
|---|---|---|---|---|---|
| Escherichia coli | $5.0\times10^6$ | $3\times10^4$ | <10 | <10 | <10 |
| Pseudomonas aeruginosa | $5.0\times10^6$ | $3\times10^4$ | <10 | <10 | <10 |
| Staphylococcus aureus | $5.0\times10^6$ | $2\times10^4$ | <10 | <10 | <10 |
| Candida albicans | $5.0\times10^5$ | $3\times10^4$ | $1.5\times10^3$ | <10 | <10 |
| Aspergillus brasiliensis | $5.0\times10^5$ | $3\times10^4$ | 500 | <10 | <10 |

On the basis of the results obtained, the niosome dispersion (CBD-S5) of example 1 passed the challenge test, showing inhibitory activity against all microorganisms according to the acceptability criteria that provide for a 99.9% reduction of bacteria and 90% of moulds or fungi inoculated within 7 days of inoculation, and a further reduction in the subsequent period (CTFA M-3 and M4, ISO 11930:2012, and Ph Eu 5.3.1).

**EXAMPLE 7** - **Stability testing by accelerated ageing**

The niosome dispersion (CBD-S5) of Example 1 was subjected to a series of stability and accelerated ageing tests under different conditions:

(A) Room temperature (25°C) with exposure to light for 90 days
(B) Temperature of 40°C for 90 days
(C) Temperature of 4°C for 90 days
(D) Thermal shock: 15 days at 4°C and 15 days at 40°C (3 cycles)

The parameters of interest (appearance, color, pH, density) were monitored bi-weekly for a total period of 90 days.
The results are summarized in the following Table 9.

TABLE 9

| Storage A : 25°C with light for 90 days | | | | | | |
|---|---|---|---|---|---|---|
| | 15 days | 30 days | 45 days | 60 days | 75 days | 90 days |
| pH | 5.25 | 5.24 | 5.25 | 5.22 | 5.23 | 5.21 |
| DENSITY | 1.081 | 1.080 | 1.080 | 1.078 | 1.079 | 1.079 |
| COLOR | UNCHANGED | LIGHT RED | DARK RED | DARK RED | DARK RED | DARK RED |
| APPEARANCE | UNCHANGED | UNCHANGED | UNCHANGED | UNCHANGED | UNCHANGED | UNCHANGED |

| Storage B : 40°C for 90 days | | | | | | |
|---|---|---|---|---|---|---|
| | 15gg | 30gg | 45gg | 60gg | 75gg | 90gg |
| pH | 5.24 | 5.23 | 5.23 | 5.21 | 5.22 | 5.22 |
| DENSITY | 1.080 | 1.081 | 1.080 | 1.082 | 1.081 | 1.083 |
| COLOR | UNCHANGED | UNCHANGED | UNCHANGED | UNCHANGED | LIGHT RED | LIGHT RED |
| APPEARANCE | UNCHANGED | UNCHANGED | UNCHANGED | UNCHANGED | UNCHANGED | UNCHANGED |

| Storage C : 4°C for 90 days | | | | | | |
|---|---|---|---|---|---|---|
| | 15gg | 30gg | 45gg | 60gg | 75gg | 90gg |
| pH | 5.23 | 5.22 | 5.19 | 5.17 | 5.17 | 5.15 |
| DENSITY | 1.081 | 1.082 | 1.084 | 1.083 | 1.085 | 1.086 |
| COLOR | UNCHANGED | UNCHANGED | LIGHT RED | LIGHT RED | DARK RED | DARK RED |
| APPEARANCE | UNCHANGED | UNCHANGED | UNCHANGED | UNCHANGED | UNCHANGED | UNCHANGED |

| Storage D : 15 days at 4°C and 15 days at 40 °C (3 cycles) | | | | | | |
|---|---|---|---|---|---|---|
| | 15gg | 30gg | 45gg | 60gg | 75gg | 90gg |
| pH | 5.25 | 5.22 | 5.20 | 5.21 | 5.20 | 5.10 |
| DENSITY | 1.081 | 1.080 | 1.079 | 1.083 | 1.082 | 1.084 |
| COLOR | UNCHANGED | UNCHANGED | LIGHT RED | LIGHT RED | DARK RED | DARK RED |
| APPEARANCE | UNCHANGED | UNCHANGED | UNCHANGED | UNCHANGED | UNCHANGED | UNCHANGED |

After 90 days of accelerated ageing under various stressful conditions, the product parameters did not change significantly, in particular the density as an index of stability of the niosomal vesicle, with the exception of the color which changed from yellow to red. This change is due to the known instability of cannabidiol to heat and light. Storage conditions for the niosomal preparation include packaging that is impermeable to light and air and maintained at room temperature.

## EXAMPLE 8 - Particulate resistance and cell viability test

The test involves treating Reconstituted Human Epidermis (RHE) tissue, whether or not protected with the niosome dispersion (CBD-S5) of Example 1, with a particulate sample with an aerodynamic diameter of less than 2.5 $\mu$m (PM2.5) chosen to be representative of ambient particulate pollution. In parallel, the reconstituted human epidermis tissue was treated with Dulbecco's phosphate buffered saline (DPBS - negative control NC), sodium dodecyl sulphate (SDS - positive control PC), and niosome dispersion alone (CBD-S5) from Example 1.

Specifically, wells containing RHE (EpiDerm™ EPI-200, MakTek Corporation) in their own culture medium (EpiDerm™ EPI-100-NMM, MakTek Corporation) treated with (1) 30$\mu$l DPBS (NC), (2) 30$\mu$l SDS 5% (PC), (3) 20$\mu$g PM2.5 dissolved in 30$\mu$l PBS, (4) 30$\mu$l CBD-S5, and (5) 30$\mu$l CBD-S5 with 20$\mu$g PM2.5 have been set up in triplicate.

After incubation at 37°C for 24 h in a controlled atmosphere of 5% $CO_2$, the cell viability of the RHE sample was assessed by MTT assay, as described in Example 5A, while the culture medium was collected and analyzed for IL-1$\alpha$ release by ELISA assay, using an EMIL1ALPHA kit (Invitrogen-Thermo Scientific) with the following procedure.

100 $\mu$l of medium from each treatment was transferred to wells coated with anti-human IL-1$\alpha$ and incubated for 2 hours at room temperature. At the end of the incubation, the medium was removed and the wells were washed 4 times with 1X Wash Buffer. 100$\mu$l of biotinylated antibody was added to each well and incubated for 1 hour at room temperature. At the end, the wells were washed 4 times with 1X Wash Buffer, treated with 100 $\mu$l of Streptavidin-HRP solution for 45 minutes at room temperature and finally washed 4 times with 1X Wash Buffer. 100 $\mu$l of TMB substrate was added to each well and incubated for 10 minutes in the dark. Finally, 100 $\mu$l of stop solution was added to each well and spectrophotometric readings were taken at wavelengths of 450nm and 550nm using the $\mu$QUANT BIO TEK Micro-plate Spectrophotometer instrument.

Changes in IL-1$\alpha$ levels released from the RHE tissue after each treatment were expressed as % values compared to the untreated RHE (NC) used as control (100%).

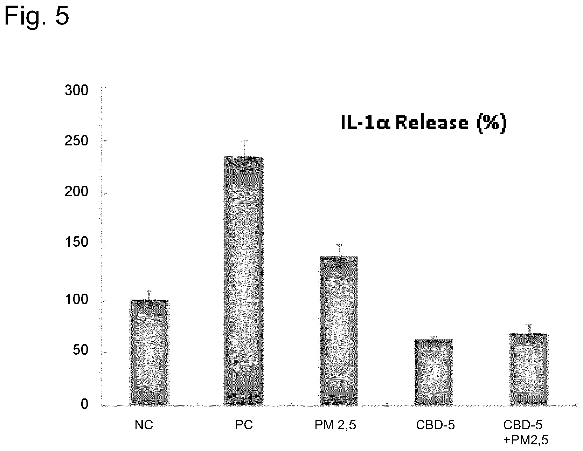

The following Table 10 summarises the results of the tests, also shown in Figures 4 and 5.

TABLE 5

| Sample | Mean viability (%) | Standard Deviation | Mean IL-1$\alpha$ release (%) | Standard Deviation |
|---|---|---|---|---|
| NC | 100.00 | 3.10 | 100.00 | 9.7 |
| PC | 7.61 | 0.60 | 235.35 | 14.3 |
| PM2.5 | 28.51 | 8.35 | 140.40 | 10.6 |
| CBD-S5 | 110.86 | 6.11 | 63.26 | 2.5 |
| CBD-S5+PM2.5 | 71.25 | 7.44 | 68.43 | 8.0 |

The in vitro MTT test showed that PM2.5 reduced cell viability to 28.51%, while co-treatment with CBD-S5 protected the reconstituted human epidermis (RHE) against the toxic effect of PM2.5. The data showed that CBD-S5 was able to increase cell viability from 28.5% to 71.25%. At the same time, the results of the CBD-S5-only test surprisingly showed an increase in cell viability over the control to 110.86%, thus indicating a surprising regenerative activity. It is believed that this increase may be due to a revitalizing action brought about by the presence of the polysaccharide beta-glucan.

The ELISA assay showed that (i) PM2.5 at a concentration of 20pg/well induced an increase in IL-1$\alpha$ release, and (ii) treatment with CBD-S5 was able to reduce both IL-1$\alpha$ release in untreated and 20pg/well PM2.5 treated tissues. Again, a surprising reduction in IL-1$\alpha$ release was observed in the sample treated with CBD-S5 alone, thus indicating significant anti-inflammatory activity.

## EXAMPLE 9 - In vitro evaluation of soothing efficacy

The assay assessed quantitatively the effects of the sample in protecting skin cells and in inhibiting the soothing reaction caused by moderate irritating agents as sodium lauryl sulfate (SLS) by means of a multilayer in vitro skin cell model.

The inhibition of the SLS-induced release of IL-1α cytokine was investigated (through specific ELISA assay) following exposure to the tested substance and respect to skin untreated samples. Cell viability was determined by the MTT assay.

Both assays used a reconstructed artificial human skin model comprising normal human epidermal keratinocytes, growing as an integrated three-dimensional cell culture model, perfectly mimicking the human skin in vitro. The model exhibits normal barrier functions (presence of a well-differentiated stratum corneum). The model was supplied by Episkin (Lion, Batch 20-RHE-041).

Epidermis was treated for 70 minutes with SLS at 0.30% in order to induce IL-1α synthesis and release. Later, 40 μl of the niosome dispersion (CBD-S5) of Example 1 (diluted at 2% in sterile water) has been applied on two epidermis and the exposure has been carried out for 24 hours at 37°C, 5% $CO_2$. As negative control, two epidermis units were treated with phosphate buffer, while epidermis units treated for 70 minutes with SLS 0.30% were used as positive control. Test was carried out in two replicates.

At the end of the exposure period, sample was removed by phosphate buffer washings (PBS) and the MTT assay was carried out to evaluate the cell survival.

Epidermis units were treated with 1 mg/ml MTT solution (3-[4,5-dimethylthiazol-2-yl]-2,5-diphenyl tetrazolium bromide) for 3 h at 37°C. The solution was then removed and replaced with isopropanol, with further 2 h incubation at room temperature. Two aliquots of every sample were transferred to a 96 well plate for the reading. The absorbance (OD) was read at the wavelength of 570 nm with a colorimeter (Tecan model Infinite F200) equipped with a microplate reader.

The results were expressed in terms of cell viability percent and % relative protection obtained with the following formulas:

$$\% \text{ cell viability (cv)} = [OD_{570} \text{ TS} / OD_{570} \text{ NC}] \times 100$$

$$\% \text{ relative protection} = (\% \text{ cv TS} - \% \text{ cv PC}) / (\% \text{ cv NC} - \% \text{ cv PC})$$

The results are summarized in the following Table 6.

TABLE 6

| Sample | % mean cell viability (standard deviation) | % relative protection |
|---|---|---|
| CBD-S5 (TS) | 73.34 (5.88) | 49.35 |
| Positive control (PC) | 47.35 (8.41) | |
| Negative control (NC) | 100.00 (0.67) | |

For IL-1α assay, the culture medium has been collected at 6 and 24 hours. As negative control, culture medium of units treated with PBS has been used; while culture medium of 2 units treated with SLS 0.30% has been used as positive control. IL-1α was determined in the medium culture of treated and not treated epidermis, using a direct ELISA test (Enzyme-linked immunosorbent assay). The colorimetric signal was directly proportional to the amount of cytokine in the culture medium. Samples were read at 450 nm. The sensitivity limit was less than 10 pg/ml.

Cytokine concentration was determined using a standard curve. The results were expressed in terms of IL-1α release inhibition percent obtained with the following formula:

$$\% \text{ IL-1α inhibition} = 100 - (\text{pg/ml IL-1α release test sample} / \text{pg/ml IL-1α positive control})*100$$

The results are summarized in the following Table 7.

TABLE 7

| Sample | IL-1$\alpha$ | | | |
|---|---|---|---|---|
| | 6 hours | | 24 hours | |
| | pg/ml | % inhibition | pg/ml | % inhibition |
| CBD-S5 | 31.47 | 70.19 | 355.30 | 40.52 |
| Positive control | 105.57 | | 597.32 | |
| Negative control | 21.26 | | 30.37 | |

The data summarized in Tables 6 and 7 demonstrated that CBD-S5 was able in protecting skin cells and inhibiting the IL-1$\alpha$ release, so showing an in-vitro soothing activity.

**EXAMPLE 10** - **Mitogenesis in hair bulb cells**

The purpose of this assay was to assess quantitatively the effects of the CBD-S5 on the stimulation of the cell cycle and on the total protein synthesis in human hair dermal papilla fibroblasts through the cell viability assay (MTT) after starving and the total protein extraction and quantification at different exposure times at sub-toxic and serial dosages. When a composition is able to increase the cell proliferation and the protein synthesis, this will suggest its role in stimulating the follicle growth and the early anagenesis phase.

The test is carried out on human hair dermal papilla cells (HHDPCs) with fibroblast-like morphology. Cells are cultured in MEM containing 10% FBS and antibiotics.

Cells have been seeded in 24 wells plates and allowed to grow for 24 h at 37°C and 5% $CO_2$. Cells underwent starvation in serum-free medium for 6 hours before treatment. Fresh medium without serum and supplemented with serial dilutions of the niosome dispersion (CBD-S5) of Example 1 (0.006 and 0.003 mg/ml) was then added to the cells. The niosome dispersion was dissolved directly in the medium culture. The test was carried out in three replicates.

After 24- and 48-hours exposure the cell viability and the total cellular protein content were assessed on separate plates. Untreated cells were used as negative controls (NC); cells treated with human insulin were used as positive control (PC).

The cell viability was assessed by incubating the tissues for 2 hours with MTT (3-[4,5-dimethylthiazol-2-yl]-2,5-diphenyl tetrazolium bromide) solution. The precipitated formazan was then extracted using DMSO and quantified spectrophotometrically. The absorbance at 570 nm ($OD_{570}$) was measured with a microplate reader (Tecan, Infinite 200 PRO), deducting background at 650 nm ($OD_{650}$).

The cell viability was expressed in percentage terms according to the following formula:

$$\% \text{ of cell viability} = [(OD_{570} - OD_{650}) \text{ test product} / (OD_{570} - OD_{650}) \text{ NC}] \times 100$$

The results are summarized in the following Table 8.

TABLE 8

| Sample | 24 hours | | 48 hours | |
|---|---|---|---|---|
| | % cell viability (ds) | p Value vs negative control | % cell viability (ds) | p Value vs negative control |
| CBD-S5 0.006 mg/ml | 89.38 (5.99) | n.s. | 117.77 (3.04) | n.s. |
| CBD-S5 0.003 mg/ml | 93.45 (13.26) | n.s. | 119.04 (5.90) | <0.05 |
| Positive control | 136.73 (19.50) | <0.05 | 136.72 (8.16) | <0.01 |
| Negative control | 100.00 (6.80) | - | 100.00 (4.09) | - |

The total cellular protein content was tested according to Bradford method. Cells at the end of the treatment were washed twice with PBS and lysed by treatment with purified water at 4°C. Dye reagent was added to each sample.

A standard curve with titrated was set with a concentration range from 2 to 12 $\mu$g/ml. 200 $\mu$l of each sample, controls and standards were transferred to a 96 wells plate. Reading was made at 595 nm with a colorimeter equipped with a plate-reader.

To quantify proteins, a standard plot with albumin was designed and the sample concentrations were determined on the bases of their absorbance values using the interpolation plot formula. Mean values of every set of data were calculated, and for each sample the percentage of increase in the protein synthesis was determined by comparison with the negative control (NC) according to the following formula:

$$\% \text{ increase in the protein synthesis} = (\mu g/ml \text{ protein sample} / \mu g/ml \text{ NC})*100$$

The results are summarized in the following Table 9.

TABLE 9

| Sample | 24 hours | | 48 hours | |
|---|---|---|---|---|
| | % protein synthesis (ds) | p Value vs negative control | % protein synthesis (ds) | p Value vs negative control |
| CBD-S5 0.006 mg/ml | 84.84 (3.96) | n.s. | 106.10 (5.27) | n.s. |
| CBD-S5 0.003 mg/ml | 88.64 (11.01) | n.s. | 126.61 (8.14) | <0.05 |
| Positive control | 140.65 (12.53) | <0.01 | 149.65 (10.83) | <0.01 |
| Negative control | 100.00 (6.29) | - | 100.00 (0.83) | - |

The data summarized in Tables 8 and 9 demonstrated that CBD-S5 was able to stimulate cells proliferation and protein synthesis in human hair dermal papilla fibroblasts compared to untreated control cell cultures (negative control) at 0.003 mg/ml concentration after a 48 hours exposure-period.

**EXAMPLE 11 - Anti-itch activity**

*In vivo,* a basophil cell degranulation mediated by IgE triggers an immediate allergic reaction characterized by the quick release of pre-stored inflammatory mediators (histamine, leukotrienes, thromboxane, basophilic enzymes, hexosaminidases), causing redness, rashes, itchiness and local inflammatory reactions. The ability of a composition to inhibit the degranulation of basophil cells is an indicator of its capability to inhibit the above described symptoms and above them mainly itching.

The test is carried out on a cell line of transfected rat basophil cells (RBL-2H3 ATCC® CRL-2256™) expressing the IgE human receptor (FcRI). Cells are kept in RPMI containing 10% FCS and 2 mM glutamine.

Untreated cells were used as negative control for the spontaneous degranulation.

A positive control consisting in cells exposed to anti-human IgE receptor antibodies was used to evaluate the maximal degranulation level.

Cell lysates in 1% Triton were also analyzed in order to evaluate the highest possible release of the analyzed molecules.

In order to evaluate the effect of the niosome dispersion (CBD-S5) of Example 1 on basophil degranulation, the sample was pre-incubated with a cell line of transfected rat basophil cells (RBL) expressing the IgE human receptor (FcRI).

The release of $\beta$-hexosaminidase during degranulation was monitored on RBL-SX38 cell supernatant added with p-nitrophenyl-N-acetyl-$\beta$-D-glucosamine (Sigma-Aldrich) in 0.1 M citrate buffer (pH 6.2) and incubated at 37°C for 120 min. The reaction was terminated using 0.1M carbonate buffer (pH 10), and the absorbance was read at 405 nm ($OD_{405}$).

The supernatant of untreated cells was used as negative control. The maximal stimulation of RBL-SX38 was evaluated on a 1% Triton X-100 lysate of the cell monolayer.

The optical density of negative and positive control and Triton X-100 lysate is reported in the following Table 10.

TABLE 10

| Sample | $OD_{405}$ |
|---|---|
| Negative control (spontaneous degranulation) | 0.068 |
| Positive control Anti-human igE receptor | 1.030 |
| Triton X-100 | 1.852 |

The niosome dispersion (CBD-S5) of Example 1 was solubilized in cell culture medium at different concentrations, as indicated in the following Table 11.

Sodium hyaluronate in serial dilutions, as indicated in the following Table 11, was also used as a control of the inhibition of basophil degranulation.

The measured optical density at 405 nm ($OD_{405}$) is reported in the following Table 11.

TABLE 11

| Hyaluronic acid (HA) + Positive control (PC) | 10 mg/ml | 5 mg/ml | 2,5 mg/ml | 1,25 mg/ml |
|---|---|---|---|---|
| | 0.255 | 0.527 | 0.690 | 0.879 |
| CBD-S5 + Positive control (PC) | 300 $\mu$g/ml | 150 $\mu$g/ml | 75 $\mu$g/ml | 32,5 $\mu$g/ml |
| | 0.844 | 0.885 | 0.960 | 1.004 |
| Hyaluronic acid (HA) | 10 mg/ml | 5 mg/ml | 2,5 mg/ml | 1,25 mg/ml |
| | 0.065 | 0.077 | 0.084 | 0.086 |
| CBD-S5 | 300 $\mu$g/ml | 150 $\mu$g/ml | 75 $\mu$g/ml | 32,5 $\mu$g/ml |
| | 0.063 | 0.085 | 0.080 | 0.093 |

The inhibition degranulation percentage of activated basophil with IgE receptor has been calculated with the following formula:

$$\% \text{ degranulation inhibition} = 100 - [(OD_{405} \text{ CBD-S5+PC} - OD_{405} \text{ NC}) / (OD_{405} \text{ PC} - OD_{405} \text{ NC})]*100$$

The results are illustrated in the following Table 12.

TABLE 12

| | % degranulation inhibition | | | |
|---|---|---|---|---|
| CBD-S5 | 300 $\mu$g/ml | 150 $\mu$g/ml | 75 $\mu$g/ml | 32,5 $\mu$g/ml |
| | 19.3 | 15.1 | 7.3 | 2.7 |
| Hyaluronic acid (HA) | 10 mg/ml | 5 mg/ml | 2,5 mg/ml | 1,25 mg/ml |
| | 80.6 | 52.3 | 35.3 | 15.7 |

The niosome dispersion (CBD-S5) was able to inhibit basophil degranulation in a dose dependent manner. The highest effect was pointed out at the highest tested concentrations. The interference on spontaneous degranulation was not detectable.

The above results demonstrated that the niosome dispersion (CBD-S5) has anti-itching activity.

## Claims

1. Topical composition comprising an aqueous-based composition comprising cannabinoids in niosomes having size lower than 500 nm, and at least one topically acceptable excipient **characterised in that** said niosomes comprise (i) at least one linear or branched polyglycerol esterified with linear saturated or monounsaturated fatty acids, and (ii) at least one polysaccharide selected from the group consisting of pullulan, glucan, alginate, amylose, glycogen, and inulin.

2.  Topical composition according to claim 1, wherein said cannabinoids are of natural or synthetic origin.

3.  Topical composition according to claim 1, wherein said cannabinoids are selected from the group consisting of cannabidiol, cannabigerol and cannabinol.

4.  Topical composition according to claim 1, wherein said polysaccharide is selected from the group consisting of alpha-glucan and beta-glucan, preferably beta-glucan.

5.  Topical composition according to claim 1, wherein said linear or branched polyglycerol is selected from the group consisting of triglycerol, tetraglycerol, hexaglycerol, octaglycerol, decaglycerol.

6.  Topical composition according to claim 1, wherein said linear saturated or monounsaturated fatty acids are selected from the group consisting of monocarboxylic acids having from 4 to 32 carbon atoms.

7.  Topical composition according to claim 1, wherein said linear saturated fatty acids are selected from the group consisting of butyric acid, valeric acid, caproic acid, enanthic acid, caprylic acid, pelargonic acid, capric acid, lauric acid, myristic acid, palmitic acid, margaric acid, stearic acid, arachidic acid, behenic acid, lignoceric acid, cerotic acid, montanic acid, melissic acid, and lacceroic acid.

8.  Topical composition according to claim 1, wherein said linear monounsaturated fatty acids are selected from the group consisting of myristoleic acid, palmitoleic acid, oleic acid, gadoleic acid and erucic acid.

9.  Topical composition according to claim 1, wherein said niosomes comprise (iii) at least one glycol having from 4 to 16 carbon atoms.

10. Topical composition according to claim 9, wherein said glycol having from 4 to 16 carbon atoms is selected from the group consisting of 1,2-butanediol, 1,2-pentanediol, 1,2-hexanediol, 1,2-heptanediol, 1,2-octanediol (caprylyl glycol), 1,2-decanediol (capryl glycol), 1,2-dodecanediol (lauryl glycol), and 1,2-hexadecanediol.

11. Aqueous-based composition comprising cannabidiol incorporated in niosomes, **characterized in that** said niosomes comprise (i) at least one linear or branched polyglycerol esterified with linear saturated or monounsaturated fatty acids, and (ii) at least one polysaccharide selected from the group consisting of pullulan, glucan, alginate, amylose, glycogen, and inulin, and, optionally, (iii) at least one glycol having from 4 to 16 carbon atoms.

12. Composition according to claim 11, wherein said composition is an aqueous solution or dispersion.

13. A method for manufacturing of an aqueous-based composition as defined in claim 11 or 12, comprising the use of hand shaking techniques or ultrasonic shaking techniques.

**Patentansprüche**

1.  Topische Zusammensetzung, die eine Zusammensetzung auf wässriger Basis umfasst, die Cannabinoide in Niosomen mit einer Größe von weniger als 500 nm und mindestens einen topisch annehmbaren Hilfsstoff umfasst, **dadurch gekennzeichnet, dass** die Niosomen (i) mindestens ein lineares oder verzweigtes Polyglycerin, das mit linearen gesättigten oder einfach ungesättigten Fettsäuren verestert ist, und (ii) mindestens ein Polysaccharid, das aus der Gruppe ausgewählt ist, die aus Pullulan, Glucan, Alginat, Amylose, Glykogen und Inulin besteht, umfassen.

2.  Topische Zusammensetzung nach Anspruch 1, wobei die Cannabinoide natürlichen oder synthetischen Ursprungs sind.

3.  Topische Zusammensetzung nach Anspruch 1, wobei die Cannabinoide aus der Gruppe ausgewählt sind, die aus Cannabidiol, Cannabigerol und Cannabinol besteht.

4.  Topische Zusammensetzung nach Anspruch 1, wobei das Polysaccharid aus der Gruppe ausgewählt ist, die aus Alpha-Glucan und Beta-Glucan besteht, vorzugsweise Beta-Glucan.

**5.** Topische Zusammensetzung nach Anspruch 1, wobei das lineare oder verzweigte Polyglycerin aus der Gruppe ausgewählt ist, die aus Triglycerin, Tetraglycerin, Hexaglycerin, Octaglycerin und Decaglycerin besteht.

**6.** Topische Zusammensetzung nach Anspruch 1, wobei die linearen gesättigten oder einfach ungesättigten Fettsäuren aus der Gruppe ausgewählt sind, die aus Monocarbonsäuren mit 4 bis 32 Kohlenstoffatomen besteht.

**7.** Topische Zusammensetzung nach Anspruch 1, wobei die linearen gesättigten Fettsäuren aus der Gruppe ausgewählt sind, die aus Buttersäure, Valeriansäure, Capronsäure, Enanthsäure, Caprylsäure, Pelargonsäure, Caprinsäure, Laurinsäure, Myristinsäure, Palmitinsäure, Margarinsäure, Stearinsäure, Arachidinsäure, Behensäure, Lignocerinsäure, Cerotinsäure, Montansäure, Melissinsäure und Laccerinsäure besteht.

**8.** Topische Zusammensetzung nach Anspruch 1, wobei die linearen einfach ungesättigten Fettsäuren aus der Gruppe ausgewählt sind, die aus Myristoleinsäure, Palmitoleinsäure, Ölsäure, Gadoleinsäure und Erucasäure besteht.

**9.** Topische Zusammensetzung nach Anspruch 1, wobei die Niosomen (iii) mindestens ein Glykol mit 4 bis 16 Kohlenstoffatomen umfassen.

**10.** Topische Zusammensetzung nach Anspruch 9, wobei das Glykol mit 4 bis 16 Kohlenstoffatomen aus der Gruppe ausgewählt ist, die aus 1,2-Butandiol, 1,2-Pentandiol, 1,2-Hexandiol, 1,2-Heptandiol, 1,2-Octandiol (Caprylylglykol), 1,2-Decandiol (Caprylglykol), 1,2-Dodecandiol (Laurylglykol) und 1,2-Hexadecandiol besteht.

**11.** Zusammensetzung auf wässriger Basis, die Cannabidiol umfasst, das in Niosomen enthalten ist, **dadurch gekennzeichnet, dass** die Niosomen (i) mindestens ein lineares oder verzweigtes Polyglycerin, das mit linearen gesättigten oder einfach ungesättigten Fettsäuren verestert ist, und (ii) mindestens ein Polysaccharid, das aus der Gruppe ausgewählt ist, die aus Pullulan, Glucan, Alginat, Amylose, Glykogen und Inulin besteht, und gegebenenfalls (iii) mindestens ein Glykol mit 4 bis 16 Kohlenstoffatomen umfassen.

**12.** Zusammensetzung nach Anspruch 11, wobei die Zusammensetzung eine wässrige Lösung oder Dispersion ist.

**13.** Verfahren zur Herstellung einer Zusammensetzung auf wässriger Basis, wie in Anspruch 11 oder 12 definiert, umfassend die Verwendung von Handschütteltechniken oder Ultraschallschütteltechniken.


**Revendications**

**1.** Composition topique comprenant une composition à base aqueuse comprenant des cannabinoïdes dans des niosomes ayant une taille inférieure à 500 nm, et au moins un excipient topiquement acceptable **caractérisée en ce que** lesdits niosomes comprennent (i) au moins un polyglycérol linéaire ou ramifié estérifié avec des acides gras linéaires saturés ou monoinsaturés, et (ii) au moins un polysaccharide sélectionné dans le groupe constitué du pullulane, glucane, de l'alginate, de l'amylose, du glycogène, et de l'inuline.

**2.** Composition topique selon la revendication 1, lesdits cannabinoïdes étant d'origine naturelle ou synthétique.

**3.** Composition topique selon la revendication 1, lesdits cannabinoïdes étant sélectionnés dans le groupe constitué du cannabidiol, du cannabigérol et du cannabinol.

**4.** Composition topique selon la revendication 1, ledit polysaccharide étant sélectionné dans le groupe constitué de l'alpha-glucane et du bêta-glucane, préférablement du bêta-glucane.

**5.** Composition topique selon la revendication 1, ledit polyglycérol linéaire ou ramifié étant sélectionné dans le groupe constitué du triglycérol, tétraglycérol, de l'hexaglycérol, de l'octaglycérol, du décaglycérol.

**6.** Composition topique selon la revendication 1, lesdits acides gras linéaires saturés ou monoinsaturés étant sélectionnés dans le groupe constitué des acides monocarboxyliques ayant de 4 à 32 atomes de carbone.

**7.** Composition topique selon la revendication 1, lesdits acides gras linéaires saturés étant sélectionnés dans le groupe constitué de l'acide butyrique, acide valérique, acide caproïque, acide énanthique, acide caprylique, acide pélargonique, acide caprique, acide laurique, acide myristique, acide palmitique, acide margarique, acide stéarique, acide arachidique, acide béhénique, acide lignocérique, acide cérotique, acide montanique, acide mélissique, et de l'acide laccéroïque.

8. Composition topique selon la revendication 1, lesdits acides gras linéaires monoinsaturés étant sélectionnés dans le groupe constitué de l'acide myristoléique, acide palmitoléique, acide oléique, acide gadoléique et de l'acide érucique.

9. Composition topique selon la revendication 1, lesdits niosomes comprenant (iii) au moins un glycol ayant de 4 à 16 atomes de carbone.

10. Composition topique selon la revendication 9, ledit glycol ayant de 4 à 16 atomes de carbone étant sélectionné dans le groupe constitué du 1,2-butanediol, 1,2-pentanediol, 1,2-hexanediol, 1,2-heptanediol, 1,2-octanediol (caprylyl glycol), 1,2-décanediol (capryl glycol), 1,2-dodécanediol (lauryl glycol), et du 1,2-hexadécanediol.

11. Composition à base aqueuse comprenant du cannabidiol incorporé dans des niosomes, **caractérisée** en que lesdits niosomes comprennent (i) au moins un polyglycérol linéaire ou ramifié estérifié avec des acides gras linéaires saturés ou monoinsaturés, et (ii) au moins un polysaccharide sélectionné dans le groupe constitué du pullulane, glucane, de l'alginate, de l'amylose, du glycogène, et de l'inuline, et, éventuellement, (iii) au moins un glycol ayant de 4 à 16 atomes de carbone.

12. Composition selon la revendication 11, ladite composition étant une solution ou une dispersion aqueuse.

13. Procédé de fabrication d'une composition à base aqueuse telle que définie selon la revendication 11 ou 12, comprenant l'utilisation de techniques d'agitation manuelle ou de techniques d'agitation par ultrasons.

Fig. 1

Fig. 2

Fig. 3

Fig. 4

Fig. 5

**EP 4 114 355 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019023668 A1

- WO 2017168354 A1

**Non-patent literature cited in the description**

- **ABRAMS, D.I.** *European Journal of Internal Medicine,* March 2018, vol. 49, 7-11
- **BABALONIS et al.** *Drug Alcohol Depend.,* 01 March 2017, vol. 172, 9-13
- **PISANTI et al.** Cannabidiol: State of the art and new challenges for therapeutic applications. *Pharmacology and Therapeutics,* July 2017, vol. 175, 133-150
- **E. RIPAMONTI et al.** Evaluation of the efficacy of proprietary niosomes as enhancers of skin permeability of plant extracts: an in vitro study. *H&PC Today,* March 2018, vol. 13 (2
- **MADHAV et al.** Niosomes: a novel drug delivery system. *International journal of research in pharmacy and chemistry,* 2011, vol. 1 (3), 498-5.11